Europäisches Patentamt

European Patent Office

Office européen des brevets

(11) Publication number: **0 160 553**
**B1**

(12) **EUROPEAN PATENT SPECIFICATION**

(45) Date of publication of patent specification: **19.04.89**

(51) Int. Cl.⁴: **C 07 C 15/46, C 07 C 7/04**

(21) Application number: **85302996.5**

(22) Date of filing: **26.04.85**

(54) Process for distillation of styrenes.

(30) Priority: **26.04.84 JP 82863/84**
**22.08.84 JP 174702/84**
**24.08.84 JP 176392/84**
**28.12.84 JP 278874/84**

(43) Date of publication of application:
**06.11.85 Bulletin 85/45**

(45) Publication of the grant of the patent:
**19.04.89 Bulletin 89/16**

(84) Designated Contracting States:
**DE FR GB IT NL**

(56) References cited:
**US-A-4 395 310**

(73) Proprietor: **NIPPON STEEL CHEMICAL CO. LTD.**
**13-16 Ginza 5-chome Chuo-ku**
**Tokyo 104 (JP)**

(73) Proprietor: **HITACHI, LTD.**
**6, Kanda Surugadai 4-chome Chiyoda-ku**
**Tokyo (JP)**

(72) Inventor: **Horigome, Tsukumo**
**4462-2, Aza-Daimaruo Oaza-Yokoo**
**Oita-shi Oita-ken (JP)**
Inventor: **Tsuzuki, Fusayuki**
**69-25, Mita**
**Nagareyama-shi Chiba-ken (JP)**
Inventor: **Shimada-Kenji**
**Second Showa Housing Development 2668,**
**Oaza-Kawach**
**Kudamatsu-shi Yamaguchi-ken (JP)**
Inventor: **Shibuya, Masahiro**
**4-6, Minami-cho**
**Hachioji-Shi Tokyo (JP)**
Inventor: **Hakutoh, Norimasa**
**21-2, Nisiyama 2-chome**
**Kashiwa-shi Chiba-ken (JP)**
Inventor: **Kawabe, Noriyuki**
**422-6, Oaza-Minaharu**
**Oita-shi Oita-ken (JP)**

Courier Press, Leamington Spa, England.

**EP 0 160 553 B1**

(72) Inventor: **Takiue, Mamoru**
**156-135, Oaza-Yamada Okagaki-cho**
**Onga-gun Fukuoka-ken (JP)**
Inventor: **Imai, Kunihiko**
**274-196, Oaza-ota**
**Iwatsuki-shi Saitama-ken (JP)**
Inventor: **Uchiyama, Noriaki**
**7-6, Tamagawa 3-chome**
**Chofu-shi Tokyo (JP)**
Inventor: **Watanabe, Chiaki**
**11-7, Wakamiya 3-chome**
**Ichikawa-chi Chiba-ken (JP)**
Inventor: **Abe, Junichi**
**11-7, Wakamiya 3-chome**
**Ichikawa-shi Chiba-ken (JP)**

(74) Representative: **Cockbain, Julian et al**
**Frank B. Dehn & Co. Imperial House 15-19,**
**Kingsway**
**London WC2B 6UZ (GB)**

## Description

The present invention relates to a process for the distillation of styrenes by heat pump system.

For distillation, heating for vaporizing liquid and cooling for condensing the vaporized vapor are required. The amount of heat for vaporization is approximately the same as that for condensation. Particularly for the purpose of improving the efficiency of distillation operation, it is important to decrease the amount of heat required for vaporization. For decreasing the amount of heat used in distillation columns, processes for distillation by the heat pump system are known (see U.S. Patent 4,395,310, U.S. Patent 4,056,444, Published Unexamined Japanese Patent Application 111,466/77, Published Examined Japanese Patent Application 39,236/79).

These processes for distillation comprise adiabatic compression of vapor of low boiling point components discharged from the top of a distillation column with a compressor to elevate temperature and utilize the latent heat of the vapor as a heat source for a reboiler. In these processes, the vapor from the top can be utilized as a heat source by externally adding a small quantity of energy. For this reason, these processes for distillation are said to be extremely useful from a viewpoint of thermal economy, as compared to ordinary distillation system.

Now, styrenes such as styrene, vinyltoluenes, α-methylstyrene, etc. are prepared by dehydrogenation of the corresponding ethylbenzenes such as ethylbenzene, ethyltoluenes, cumene, etc. These dehydrogenated oils (crude styrenes) are mainly composed of the resulting styrenes and the unreacted ethylbenzenes, which contain small quantities of light components such as benzene, toluene, etc. and heavy components such as styrene oligomers. Accordingly, it is necessary to separate and remove the light components and heavy components from the aforesaid dehydrogenated oils, upon preparation of styrenes. As such a process, a process for distillation using a plurality of distillation columns has been adopted.

However, the boiling points of styrenes are closely akin to those of ethylbenzenes. For example, in the case of styrene and ethylbenzene, the boiling point of the former is 145.2°C and the latter is 136.2°C, under atmospheric pressure. The difference in boiling point therebetween is merely 9.0°C. Therefore, in order to separate styrenes from ethylbenzenes by distillation, a high efficiency of distillation is required and it is thus necesssry to increase the number of stages of distillation columns and a reflux ratio. For this reason, it is general that trays of 60 to 100 stages are set in a distillation column used for distillation of styrenes, in case that the column is a multi-stage column. It is also general that the reflux ratio is set to a considerably large volume of approximately 10. Thus, the pressure difference between the column top and bottom is large, e.g. 90 to 250 mmHg (12.0 to 33.3 kPa). In such a distillation column, therefore, a problem arises in that the energy to be supplied as a heat source becomes too large a quantity.

It would thus be desirable to adopt the process for distillation by the aforesaid heat pump system as a process for distillation of styrenes. However, when the difference in temperature between the top and the bottom in the distillation column becomes large, it is necessary to increase a compression ratio of vapor of low boiling point components. Accordingly, it has not been considered that the process for distillation using this heat pump system could be adopted for the distillation of styrene compositions containing components having similar boiling points and polymerizable components.

An object of the present invention is to provide a process for the distillation of styrene compositions involving distillation using the heat pump system and capable of stable operation.

In one aspect the present invention provides process for the distillation of a liquid styrene composition comprising styrenes and ethylbenzenes to separate said styrenes and said ethylbenzenes, wherein said process comprises the steps of:

(a) feeding said liquid styrene composition into a middle portion of a distillation column of packed column type operated under a reduced pressure;

(b) withdrawing from the top of said distillation column vapor of low boiling point components mainly composed of ethylbenzenes and withdrawing from the bottom of said column liquid of high boiling point components mainly composed of styrenes;

(c) leading a part of said vapor of low boiling point components through a pre-heater to a compressor to elevate the temperature thereof by adiabatic compression and leading the remaining part of said vapor of low boiling components to a condenser;

(d) introducing compressed vapor from said compressor to a reboiler of said distillation column wherein at least part of said vapor is condensed and the latent heat thereby released is used as a heat source of said reboiler;

(e) leading the liquid of low boiling components condensed in said reboiler and the vapour of low boiling components uncondensed in said reboiler to a vapor-liquid separation tank and separating condensed liquid from uncondensed vapor;

(f) leading the uncondensed vapor of low boiling components from said separation tank to said condenser;

(g) leading the condensed liquid of low boiling components from said separation tank to said pre-heater there to undergo heat exchange with the vapor of low boiling components passing to said compressor;

(h) returning a part of the condensed liquid of low boiling components condensed in said condenser

3

and said reboiler to the upper portion of said distillation column and withdrawing the remaining part of said condensed liquid of low boiling components from the distillation system.

Detailed description of the invention

The present invention is directed to a process for distillation of styrenes comprising distilling a styrene composition, preferably a mixture of styrenes obtained by the dehydrogenation of ethylbenzenes, to separate styrenes from ethylbenzenes. Some or all of the low boiling point components separated out are adiabatically compressed and then condensed to provide the heat source for a reboiler. Condensed material is returned, e.g. by a recycling line, to the distillation column.

The mixture of styrenes to which the process of the present invention particularly applies is a mixture obtained by dehydrogenating ethylbenzenes and is mainly composed of the resulting styrenes and the unreacted ethylbenzenes, which normally contain small quantities of light components such as for example benzene and toluene and heavy components such as for example styrene oligomers, polymerization inhibitors, moisture, air and nitrogen. The mixtures of styrenes include not only the dehydrogenated compounds obtained by conventional dehydrogenation with steam but also those obtained by oxidative dehydrogenation in gaseous phase or liquid phase. The term "styrenes" is used to refer to compounds containing an ethenylbenzene structure such as for example styrene, vinyltoluenes and α - methylstyrene and the term "ethylbenzenes" is used to refer to compounds containing an ethylbenzene structure such as for example ethylbenzene, ethyltoluenes and isopropylbenzene.

As modes for formation of distillation columns for distilling the mixture of styrenes, there are, for example, a mode in which light components having boiling points lower than ethylbenzenes are separated in a first column, then ethylbenzenes are separated from styrenes and heavy components having boiling points higher than those in a second column and further styrenes are separated from heavy components in a third column; a mode in which in a first column ethylbenzenes and light components having boiling points lower than those are withdrawn from the top, and from the bottom, styrenes and heavy components having boiling points higher than those are separately withdrawn, and then the top distillate and the bottom distillate are fed in a second column, respectively, wherein light components are separated from ethylbenzenes with respect to the above-mentioned top distillate and with respect to the bottom distillate, styrenes are separated from heavy components. The present invention is applicable to any of the modes described above. Preferably, the distillation column of step (a) of the process of the present invention is the second column in the former case or the first column in the latter case. Accordingly, the mixtures of styrenes to which the present invention is applied may be mixtures obtained immediately after dehydrogenation of ethylbenzenes or mixtures obtained by separating and removing light components such as benzene, toluene, having boiling points lower than ethylbenzenes in advance.

The distillation column referred to in step (a) of the process of the present invention is a distillation column of packed column type which is operated under a reduced pressure. As packings to be packed in the distillation column, regular packings are preferred; packings having an average pressure difference of below 1.5 mmHg (200 Pa), preferably below 0.8 mmHg (107 Pa), for each column stage are especially preferred. By the use of such a distillation column, distillation efficiency becomes excellent and the reflux ratio can be reduced. Accordingly, the pressure difference in the distillation column can be reduced to less than 100 mmHg (13.3 kPa), preferably less than 70 mmHg (9.3 kPa). As a result, the bottom temperature can be lowered and the temperature difference between the top and the bottom can be reduced.

In the above-described distillation column, vapor of low boiling point components (mainly composed of ethylbenzenes) is withdrawn from the top and at the same time, liquid of high boiling point components (mainly composed of styrenes) is withdrawn from the bottom. A part of the vapor of low boiling point components described above which was withdrawn from the top of the distillation column is led to a compressor. By adiabatic compression with this compressor, the temperature is elevated and the thus temperature elevated vapor of low boiling point components is led to a reboiler of the distillation column. In the reboiler, a part or whole of the vapor is condensed. By doing so, the vapor is used as a heat source of the reboiler.

The fraction of the vapor of low boiling point components that is led to the compressor and the fraction of the compressed vapor that is condensed in the reboiler are selected taking into consideration the composition of styrene mixtures fed in the distillation column, composition and quantity of vapor of low boiling point components withdrawn from the top of the distillation column, efficiency of compressor, energy added with this compressor, amount of heat required for reboiler of the distillation column. With respect to the vapor of low boiling components distilled out of the top, a line for directly leading to a condenser is provided, by which a part of the vapor may be directly introduced into the condensor without passing through the compressor so as to keep thermal balance in order to stabilize the distillation system. In the reboiler, all of the vapor may be condensed when light components such as benzene, toluene, having boiling points lower than ethylbenzenes are not present in the vapor. However, where light components are present it is preferred that some of the vapor remains uncondensed. It is possible to determine the fraction to be condensed by controlling the pressure of the reboiler. When the pressure is increased, a larger fraction of the vapor can be condensed. However, markedly increased pressure results in increase in load on the compressor. Some, suitably all, of the low boiling point components condensed in the reboiler and a part of the uncondensed vapor are returned by a recycle line to the distillation column and the remainder is discharged from the distillation column. However at least part of the condensed low boiling point components are used for preheating the vapor of low boiling point components fed into the compressor since the condensed components are nonetheless still at high temperatures.

In the process of the present invention, it is preferred that raw materials for distillation or low boiling point components be charged in the distillation column prior to the start up of distillation. The precharged column may be heated with an auxiliary reboiler using an external heat source to start up distillation in a total refluxing state. For such a start up to the distillation process it is also preferred that vapor of low boiling point components distilled out of the top of the distillation column be preheated by using the bottom liquid as a heat source and that a recycling line for recycling the vapor of low boiling point components, a preheater, a compressor and a vapor-liquid separation tank be previously warmed up by the thus preheated vapor of low boiling point components; then the temperature and pressure conditions of respective parts may be brought approximately to the stationary operation conditions of the distillation column whereafter the vapor of low boiling point components distilled out of the top may be withdrawn and the column moved into normal operation.

The materials to be charged in the bottom of the distillation column upon the starting up of distillation may be a styrene mixture to be distilled but preferably is a liquid comprising low boiling point components and having a boiling point approximately the same as those of the low boiling point components contained therein. Where a liquid of low boiling point components is previously charged, the liquid of low boiling point components may contain a single component or a mixture of two or more components as far as they do not have property of polymerization and have different boiling points. As the liquid of low boiling point components, low boiling point components contained in styrene mixtures and withdrawn from the top of the distillation column in distillation are preferably used. The low boiling point components in the styrene mixture are mainly composed of ethylbenzenes and preferably contain less than 5 wt% of styrenes.

It is preferred that the reboiler pressure be set by control means on the basis of input data regarding the composition of the styrene mixture fed into the distillation column and of the temperature within the column. Forward control of the process can thus be achieved particularly advantageously by continuously monitoring the temperature at at least one location within the column and the composition of the styrene mixture, analysing the resulting data and adjusting the pressure in a vapor-liquid separation tank mounted at the exit of the reboiler accordingly.

The location of a thermometer which is provided for detecting the internal temperature of the distillation column is determined depending upon the kind of styrene mixture and the properties of the distillation column. In addition, the quantity of low boiling point components to be withdrawn or the amount of heat transfer to the reboiler, preferably both of them, can be cascade-controlled to the above-mentioned internal temperature of the distillation column such that the internal temperature of the distillation column is controlled to a set temperature. For controlling the amount of heat transfer to the reboiler, control can be effected by, in combination, a gauge for detecting pressure in the vapor-liquid separation tank, which is mounted at the exit of the reboiler, and a control valve provided at a withdrawal line for withdrawing vapor from the vapor-liquid separation tank.

Brief description of the drawing

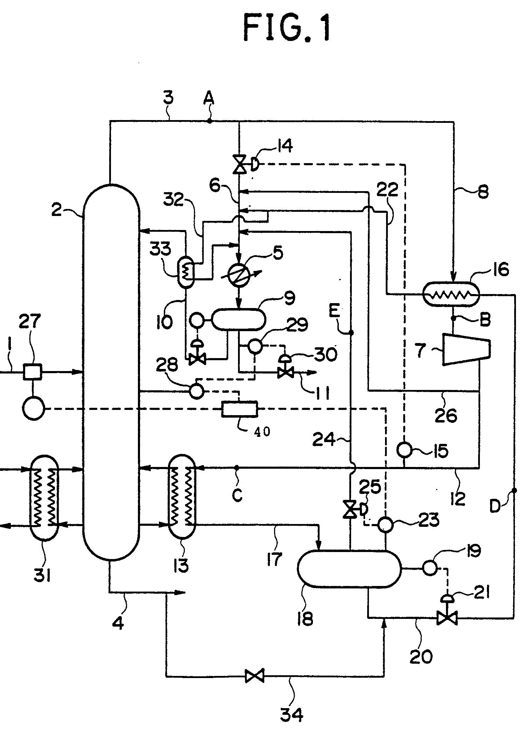

Figure 1 is a flow sheet showing a process for distillation of styrenes by heat pump system which is in accordance with the present invention.

Description of the preferred embodiments

Referring to the flow sheet shown in the drawing as an example when the present invention is applied to a styrene mixture (crude styrene) obtained by dehydrogenation of ethylbenzene, the present invention will be described in detail hereafter.

In Figure 1, the styrene mixture is fed from feeding line (1) into the middle portion of distillation column (2) of packed column type which is operated under a reduced pressure. From the top line (3) of the distillation column (2), vapor of low boiling point components mainly composed of ethylbenzene is withdrawn and, at the same time, liquid of high boiling point components mainly composed of styrene is withdrawn from line (4) at the bottom.

The vapor of low boiling point components withdrawn from line (3) is divided into recycling line (6) leading to condenser (5) and line (8) leading to compressor (7). The vapor of low boiling point components passing into line (6) is condensed by condenser (5) and thereafter fed into reflux drum (9); a part of the condensed liquid is refluxed from line (10) to the upper portion of distillation column (2) and the remaining part is withdrawn out of the system.

Further the vapor of low boiling point components passing into line (8) is adiabatically compressed to elevate its temperature to one higher than that of the bottom of the distillation column and then fed through line (12) to reboiler (13), where it is used as the heat source.

Dividing the vapor of low boiling point components into line (6) and line (8) is carried out to mainly control the quantity of the vapor of low boiling point components fed to compressor (7) to a set value and prevent from excessive heat supply to the distillation system. The quantity of the vapor divided into line (8) varies depending upon conditions for operation of the distillation system, variations in composition of raw materials, but it may be sufficient in such a quantity that the amount of heat added to the reboiler is not excessive. The fraction of the vapor that is divided into line (6) is preferably from 0.5% to 25%. As a method for control of dividing vapor, there can be adopted, for example, a method for control which comprises accumulating the condensate of the vapor of low boiling point components in condenser (5) and controlling the liquid level of the condensate with a liquid level gauge and a control valve at an outlet of the condenser to thereby increase or decrease the heat transfer surface of the condenser; a method for direct control by providing a control valve at the inlet side of condenser (5) of line (6). In the present flow sheet, control valve (14) is set in line (6) and by opening and shutting the valve in association with flow regulator (15) provided at line (12) at the outlet side of compressor (7), the quantity of the vapor of low boiling point components fed into compressor (7) is maintained at a set value.

Further if the vapor of low boiling point components fed into compressor (7) is saturated vapor, it is condensed either in the compressor or at the outlet line upon adiabatic compression by the compressor; in the compressor, liquid drops cause impeller trouble or polymerization of styrene monomer, by which serious damage is suffered in some occasions. Therefore a preheater (16) is provided on the above-mentioned line (8) and the vapor of low boiling point components fed into the compressor be preheated to elevate the temperature.

The vapor of low boiling point components which is fed into compressor (7) and charged in reboiler (13) through line (12) after elevating the temperature with adiabatic compression is used as a heat source. In reboiler (13), a part or whole of the low boiling point components is condensed and latent heat mainly generated at this stage is utilized as a heat source. The condensate or the condensate and the vapor of low boiling point components are flowed into vapor-liquid separation tank (18) via line (17), whereby the condensed low boiling point components are separated from the uncondensed vapor of low boiling point components.

In this case, in order to reduce pressure loss in reboiler (13) and also improve heat transfer efficiency, it is preferred that the line from reboiler (13) to separation tank (18) be divided into a line for flowing the condensate into separation tank (18) and a line for flowing the uncondensed vapor of low boiling point components into separation tank (18). The components condensed in this reboiler (13) are components having relatively high boiling points among the vapor of low boiling point components and mainly composed of ethylbenzene. Further the uncondensed components which pass through reboiler (13) in uncondensed state are components having relatively low boiling points among the vapor of low boiling point components and are mainly composed of benzene, toluene, moisture, air, nitrogen.

In the flow sheet, the condensed low boiling point components withdrawn from the bottom of separation tank (18) are introduced into preheater (16) while the components are controlled by liquid level gauge (19) and control valve (21) provided at line (20). After the components are heat-exchanged with the vapor of low boiling point components fed into compressor (7) by this preheater (16), the components are combined with line (6) via line (22). Preferably, the condensate is fed into heat exchanger (33) via line (32) and utilized for maintaining the reflux returned to distillation column (2) at a constant temperature since the condensate is still at a high temperature. Further the vapor of low boiling point components withdrawn from the upper portion of separation tank (18) is withdrawn while being controlled with pressure gauge (23) provided on the separation tank and pressure control valve (25) provided in line (24) and combined with line (6) through this line (24). By controlling the vapor of low boiling point components in reboiler (13) so as not to completely condense in such a manner, components having relatively high boiling points in the vapor of low boiling point components can be selectively condensed and the condensation temperature in reboiler (13) becomes high. Further, the heat transfer per unit condensation amount can be increased. In addition, the compression ratio of compressor (7) can be reduced so that running costs can be minimized and the costs for equipment can be reduced. Furthermore, there is an advantage that the temperature of the low boiling point components condensed in this reboiler (13) can be maintained at a temperature necessary for and sufficient for heating the vapor of low boiling point components in preheater (16). Control of the condensation amount of the vapor of low boiling point components in the reboiler can be performed by controlling the pressure of the reboiler, namely, the pressure of separation tank. For example, when the pressure is increased, a larger amount of the vapor is condensed and the heat transfer amount also increases; alternatively, when the pressure is decreased, the heat transfer amount can be reduced or the compression pressure can be reduced.

The variation of the vapor of low boiling point components fed into compressor (7) directly influences the variation of the vapor of low boiling point components fed into reboiler (13). Accordingly, in order to stably operate the distillation system, a control valve (not shown) having good accuracy may be provided at by-pass line (26) from compressor (7) to condenser (5) to thereby absorb minute variation of heat capacity incapable of being controlled by compressor (7).

For driving compressor (7), there are various methods such as a motor alone, combination of a motor and a steam turbine, a steam turbine alone. However, it is preferred that a system for accurately controlling rotating speed be provided in a driving machine of compressor (7) in order to achieve stabilization of the distillation system. As the mechanism for controlling the rotating speed, it is possible to adopt a VVVI (variable voltage variable frequency) control system, a VV (variable voltage) control system. For variation of voltage or frequency of an electric source or variation in pressure of steam, a feedback control system is incorporated to control the rotating speed, namely, capacity variation.

In order to stably operate the distillation system, the following control is additionally carried out in this example.

Namely, gas chromatography (27) which continuously analyzes the composition of components of the styrene mixture fed into distillation column (2) is provided at feeding line (1), at the middle portion of distillation column (2) thermometer (28) for detecting the internal temperature of the column is provided, and flow meter (29) for detecting a flow amount of the vapor of low boiling point components withdrawn and control valve (30) for controlling the flow amount are provided at withdrawal line (11) from reflux drum (9).

Further, the internal temperature of the column detected with thermometer (28), the withdrawn quantity detected by flow meter (29) and the vapor pressure of separation tank (18) detected by pressure gauge (23) are cascade-controlled respectively. At the same time, the results of the analysis of gas chromatography (27) and the internal temperature of distillation column (2) detected by thermometer (28) are fed to forward operation control means (40) by an output signal from which the set pressure of the vapor of low boiling point components in separation tank (18) is controlled. The operating equation for the forward operation control means (40) is appropriately determined depending upon the kind of styrene mixture fed in distillation column (2) through feeding line (1) and the property in dynamics of the distillation column.

In this example, where the composition of the styrene mixture fed from feeding line (1) varies, when the low boiling point components increase, forward operation control works so as to control the internal temperature of the distillation column to the set value based on the analytical results and the results of measuring the internal temperature of the distillation column; by an output signal from the operation control means (40), control valve (30) is controlled so as to increase the amount of the low boiling point components withdrawn and control valve (25) is designed to be controlled so as to increase the pressure in reboiler (13) in order to raise the condensation temperature of the vapor of low boiling point components and increase the heat transfer amount in reboiler (13). Conversely when the low boiling point components decrease, control is performed contrariwise to the case where the above-mentioned low boiling point components increase.

Further where reboiler (13) alone is insufficient for the heat source of distillation column (2) or in order to supply the heat necessary for starting-up, it is desirable to provide auxiliary reboiler (31) for introducing an external heat source such as steam, to distillation column (2). Upon starting up of distillation, it is designed such that raw materials for distillation or low boiling point components are previously charged in distillation column (2) and heated with the auxiliary reboiler to start up distillation in a total refluxing state; liquid at the bottom is passed through line (34) and fed into preheater (16) via line (20) to preheat the vapor of low boiling point components distilled out of the top; recycling lines (3), (6), (8), (10), (12), (17), (20), (22) and (24), preheater (16), compressor (7), reboiler (13) and separation tank (18) are previously warmed up with the preheated vapor of low boiling point components; then the temperature and pressure at each portion are regulated to conditions closely similar to stationary running conditions of distillation column (2), thereafter the raw materials are fed in and the compressed vapor of low boiling point components is drawn off from reboiler (13) to shift to normal running.

Further in this example, in the case of the starting up of the distillation system or when the quantity of the vapor of low boiling point components is little in separation tank (18), inert gases such as nitrogen gas, may be introduced into separation tank (18) for smooth control of condensing pressure.

Example 1

Dehydrogenated oil having the composition shown in Table 1, which had been obtained by dehydrogenating ethylbenzene in accordance with the distillation system of the example shown in Figure 1, was distilled. In the distillation, a distillation column packed with regular packings (made by Sulzer Brothers Ltd., trademark "Hellapak") was used and controlled in such a manner that the pressure difference between the top and the bottom became less than 70 mmHg. (9.3 kPa). In running of the distillation column dehydrogenated oil was constantly fed and, about 40 parts of low boiling point component liquid were withdrawn from line (11) and about 60 parts of high boiling point component liquid from line (4). The flow amount, temperature and pressure of each of points A, B, C, D and E shown in the Figure 1 were controlled to show the values shown in Table 2. The compositions of the low boiling point components and the liquid withdrawn from the bottom of distillation column (2) are as shown in Table 1.

It has been confirmed that in this example, distillation of styrene can be extremely stably performed and energy consumption of compressor (7) is reduced to about 30% of the average energy consumption of the case where steam alone was employed, thus resulting in a remarkable saving in energy consumption.

TABLE 1

| Component | Dehydrogenated oil (wt%) | Top distillate (wt%) | Liquid withdrawn from the bottom (wt%) |
|---|---|---|---|
| Benzene | 0.67 | 1.67 | — |
| Toluene | 2.25 | 5.61 | — |
| Non-aromatic hydrocarbons | 0.16 | 0.34 | 0.04 |
| Ethylbenzene | 34.45 | 90.50 | 0.04 |
| Styrene | 61.88 | 1.86 | 97.61 |
| Heavy components | 0.48 | — | 2.31 |
| Water | 0.11 | — | — |

TABLE 2

| Point | Flow amount (A=100) | Temperature (°C) | Pressure in mmHg (kPa) |
|---|---|---|---|
| A | 100 | 73 | 105 (14.0) |
| B | 95 | 83 | 90 (12.0) |
| C | 95 | 124~126 | 425 (56.7) |
| D | 90 | 120 | — |
| E | 5 | 120 | 110 (14.7) |

Example 2

Distillation was performed using dehydrogenated oil having the composition shown in Table 3, instead of dehydrogenated oil of the Example 1. The compositions of the low boiling point components and the liquid withdrawn from the bottom of distillation column (2) are as shown in Table 3. The flow amount, temperature and pressure at each of points A, B, C, D and E were controlled to show the values shown in Table 4. The distillation of styrene could be performed extremely stably also in this Example 2.

TABLE 3

| Component | Dehydrogenated oil (wt%) | Top distillate (wt%) | Liquid withdrawn from the bottom (wt%) |
|---|---|---|---|
| Benzene | 0.10 | 0.21 | — |
| Toluene | 0.16 | 0.42 | — |
| Non-aromatic hydrocarbons | 0.10 | 0.22 | 0.04 |
| Ethylbenzene | 37.59 | 97.20 | 0.04 |
| Styrene | 61.50 | 2.31 | 97.63 |
| Heavy components | 0.45 | — | 2.30 |
| Water | 0.10 | — | — |

EP 0 160 553 B1

TABLE 4

| Point | Flow amount (A=100) | Temperature (°C) | Pressure in mmHg (kPa) |
|---|---|---|---|
| A | 100 | 75 | 105 (14.0) |
| B | 88 | 83 | 90 (12.0) |
| C | 88 | 125 | 425 (56.7) |
| D | 86 | 120 | — |
| E | 2 | 120 | 110 (14.7) |

Example 3

Distillation was performed in a manner similar to Example 1 except that dehydrogenated oil was fed from line (1) at a rate of 100 parts/hr, low boiling point component liquid was withdrawn from line (11) at a rate of 36 parts/hr and high boiling point component liquid was withdrawn from line (4) at a rate of 64 parts/hr. The flow amount at point A was 270 parts/hr. The flow amount, temperature and pressure at each of points A, B, C, D and E were controlled to show the values shown in Table 5. The distillation of styrene could be performed extremely stably also in this Example 3.

TABLE 5

| Point | Flow amount (A=100) | Temperature (°C) | Pressure in mmHg (kPa) |
|---|---|---|---|
| A | 100 | 70.2 | 95 (12.7) |
| B | 99.1 | 75.8 | 319 (42.5) |
| C | 97.3 | 121.3 | 392 (52.3) |
| D | 93.7 | 91.9 | — |
| E | 3.7 | 91.9 | 385 (51.3) |

Thus the present invention provides a process for distillation by heat pump system in which response of a distillation column to variations in a composition of feed liquid, variations in heat balance due to change in conditions of the system, is improved so that the distillation column can sufficiently follow the variations in a composition of feed liquid which are applicable to distillation of styrenes.

The present invention also provides a process for distillation by heat pump system which has eliminated various problems liable to occur at the starting up of the distillation in a process for distillation by this heat pump system; for example, the problems arising where a long period of time is required until the distillation system becomes stable, during which the purity of the components obtained by distillation decreases, fouling of equipment or troubles such as plagging, occur, trouble with impeller in a compressor occurs, etc.

Claims

1. A process for the distillation of a liquid styrene composition comprising styrenes and ethylbenzenes, to separate said styrenes and said ethylbenzenes, wherein said process comprises the steps of:

(a) feeding said liquid styrene composition into a middle portion of a distillation column (2) of packed column type operated under a reduced pressure;

(b) withdrawing from the top of said distillation column vapor of low boiling point components mainly composed of ethylbenzenes and withdrawing from the bottom of said column liquid of high boiling point components mainly composed of styrenes;

(c) leading a part of said vapor of low boiling point components through a pre-heater (16) to a compressor (7) to elevate the temperature thereof by adiabatic compression and leading the remaining part of said vapor of low boiling components to a condenser (5);

(d) introducing compressed vapor from said compressor (7) to a reboiler (13) of said distillation column

9

wherein at least part of said vapor is condensed and the latent heat thereby released is used as a heat source of said reboiler;

(e) leading the liquid of low boiling components condensed in said reboiler and the vapour of low boiling components uncondensed in said reboiler to a vapor-liquid separation tank (18) and separating condensed liquid from uncondensed vapor;

(f) leading the uncondensed vapor of low boiling components from said separation tank to said condenser (5);

(g) leading the condensed liquid of low boiling components from said separation tank to said pre-heater (16) there to undergo heat exchange with the vapor of low boiling components passing to said compressor (7);

(h) returning a part of the condensed liquid of low boiling components condensed in said condenser and said reboiler to the upper portion of said distillation column and withdrawing the remaining part of said condensed liquid of low boiling components from the distillation system.

2. A process as claimed in claim 1 further comprising leading a part of the compressed vapor of low boiling components from said compressor (7) to said condenser (5) the remaining part of said compressed vapor being led to said reboiler (13).

3. A process as claimed in either of Claims 1 and 2 wherein said low boiling point components condensed in said reboiler are used as a heat source of a heat exchanger (33) for maintaining a reflux returned to said distillation column at a set temperature.

4. A process as claimed in any one of Claims 1 to 3 wherein forward operation control is effected by monitoring the internal temperature at a location within said distillation column (2), continuously analysing the composition of said styrene composition fed into said distillation column (2) and, on the basis of the data signals thereby generated, regulating the pressure in said reboiler (13).

5. A process as claimed in Claim 4 comprising cascade controlling the quantity of said low boiling components withdrawn and/or the rate heat transfer to said reboiler (13).

6. A process as claimed in any one of Claims 1 to 5 wherein an auxiliary reboiler (31) is provided to introduce heat from an external source and wherein distillation start up is effected by precharging said distillation column (2) with raw materials for distillation or with low boiling point components, heating the charge of said distillation column with said auxiliary reboiler thereby to start up distillation in a total refluxing state, and using liquid drawn from the bottom of said distillation column to preheat the said vapor of low boiling components fed to said compressor (7).

7. A process as claimed in Claim 6 wherein temperature and pressure conditions within the distillation apparatus are brought approximately to desired operating levels before continuous feed of said styrene mixture into said distillation column is commenced.

8. A process as claimed in any one of Claims 1 to 7 wherein said styrene composition comprises at least one ethylbenzene and its corresponding styrene dehydrogenation product.

**Patentansprüche**

1. Verfahren zur Destillation einer flüssigen, Styrole und Ethylbenzole umfassenden Styrol - Zusammensetzung zur Trennung der Styrole von den Ethylbenzolen, wobei das Verfahren die folgenden Schritte umfaßt:

(a) Zuführen der flüssigen Styrol-Zusammensetzung in ein Mittelteil einer Destillationskolonne (2) vom Typ einer gepackten Säule, die unter vermindertem Druck betrieben wird;

(b) Abziehen des Dampfes von Komponenten mit niedrigem Siedepunkt, die hauptsächlich aus Ethylbenzolen bestehen, vom Kopf der Destillationskolonne und Abziehen einer Flüssigkeit von Komponenten mit hohem Siedepunkt, die hauptsächlich aus Styrolen zusammengesetzt sind, vom unteren Teil der Kolonne;

(c) Leiten eines Teils des Dampfes der Komponenten mit niedrigem Siedepunkt durch einen Vorheizer (16) zu einem Kompressor (7), um dessen Temperatur durch adiabatische Kompression zu erhöhen, und Leiten des übrigen Teils des Dampfes der niedrig siedenden Komponenten zu einem Kondensator (5);

(d) Einleiten von komprimiertem Dampf aus dem Kompressor (7) in einen Aufkocher (13) der Destillationskolonne, worin wenigstens ein Teil des Dampfes kondensiert wird und die latente Wärme, die dabei freigesetzt wird, als Wärmequelle des Aufkochers verwendet wird;

(e) Überführen der Flüssigkeit aus in dem Aufkocher kondensierten, niedrig siedenden Komponenten und des Dampfes aus in dem Aufkocher nicht kondensierten, niedrig siedenden Komponenten in einen Tank (18) zur Trennung von Dämpfen und Flüssigkeiten und Trennen der kondensierten Flüssigkeit von dem unkondensierten Dampf;

(f) Überführen des unkondensierten Dampfes von niedrig siedenden Komponenten aus dem Trenn-Tank in den Kondensator (5);

(g) Überführen der kondensierten Flüssigkeit aus niedrig siedenden Komponenten aus dem Trenn-Tank in den Vorheizer (16), damit diese dort einem Wärmeaustausch mit dem Dampf aus niedrig siedenden Komponenten unterliegt, die in den Kompressor (7) strömen;

(h) Rückführen eines Teils der kondensierten Flüssigkeit von niedrig siedenden Komponenten, die in dem Kondensator und dem Aufheizer kondensiert werden, zum oberen Teil der Destillationskolonne und

EP 0 160 553 B1

Abziehen des übrigen Teils der kondensierten Flüssigkeit von niedrig siedenden Komponenten von dem Destillationssystem.

2. Verfahren nach Anspruch 1, aus außerdem folgenden Schritt umfaßt: Überführen eines Teils des komprimierten Dampfes von niedrig siedenden Komponenten aus dem Kompressor (7) in den Kondensator (5), wobei der übrige Teil des komprimierten Dampfes dem Aufheizer (13) zugeführt wird.

3. Verfahren nach einem der Ansprüche 1 und 2, worin die Komponenten mit niedrigem Siedepunkt, die in dem Aufheizer kondensiert werden, als Wärmequelle eines Wärmeaustauschers (33) zur Aufrechterhaltung eines Rückflusses verwendet werden, der bei einer festgesetzten Temperatur in die Destillationskolonne zurückgeführt wird.

4. Verfahren nach einem der Ansprüche 1 bis 3, worin eine Kontrolle des Verfahrensablaufs bewirkt wird durch Überwachen der Innentemperatur an einem Punkt innerhalb der Destillationskolonne (2), kontinuierliches Analysieren der Zusammensetzung der der Destillationskolonne (2) zugeführten Styrol - Zusammensetzung und Regulieren des Drucks in dem Aufheizer (13) auf der Grundlage der dadurch erbrachten Daten-Signale.

5. Verfahren nach Anspruch 4, das den Schritt der Kaskaden-Regelung der Menge an abgezogenen, niedrig siedenden Komponenten und/oder des Betrags des Wärmeübergangs auf den Aufheizer (13) umfaßt.

6. Verfahren nach einem der Ansprüche 1 bis 5, worin ein Hilfs-Aufheizer (31) zur Zufuhr von Wärme aus einer externen Quelle vorgesehen wird und worin der Start der Destillation dadurch bewirkt wird, daß man die Destillationskolonne (2) vorab mit Rohstoffen für die Destillation oder mit Komponenten mit niedrigem Siedepunkt befüllt, die Füllung der Destillationskolonne mit Hilfe des Hilfs-Aufheizers erwärmt, um damit die Destillation bis zu einem Zustand des vollständigen Rückflusses zu starten und Verwenden der Flüssigkeit, die vom unteren Teil der Destillationskolonne abgezogen wird, zum Vorheizen des Dampfes von niedrig siedenden Komponenten, der dem Kompressor (7) zugeführt wird.

7. Verfahren nach Anspruch 6, worin die Temperaturbedingungen und Druckbedingungen innerhalb des Destillationsapparates auf Werte nahe den gewünschten Betriebswerten gebracht werden, bevor mit der kontinuierlichen Zufuhr der Styrol-Mischung in die Destillationskolonne begonnen wird.

8. Verfahren nach einem der Ansprüche 1 bis 7, worin die Styrol-Zusammensetzung wenigstens ein Ethylbenzol und sein entsprechendes Styrol - Dehydrierungsprodukt umfaßt.

## Revendications

1. Procédé de distillation d'une composition de styrène liquide, comprenant des styrènes et des éthybenzènes, pour séparer lesdits styrènes et lesdits éthylbenzènes, ledit procédé comprenant les étapes consistant à:

(a) introduire ladite composition de styrène liquide dans une partie médiane d'une colonne de distillation (2) du type colonne à garnissage fonctionnant sous une pression réduite;

(b) retirer de la tête de ladite colonne de distillation, de la vapeur de composants à bas point d'ébullition, principalement constitués par des éthylbenzènes, et retirer du bas de ladite colonne, du liquide de composants à point d'ébullition élevé, principalement constitués par des styrènes;

(c) adresser une partie de ladite vapeur de composants à bas point d'ébullition, à travers un dispositif de préchauffage (16), à un compresseur (7), pour élever sa température par compression adiabatique, et adresser la partie restante de ladite vapeur de composants à bas point d'ébullition à un condenseur (5);

(d) introduire de la vapeur comprimée provenant dudit compresseur (7) dans un rebouilleur (13) de ladite colonne de distillation, dans lequel au moins une partie de ladite vapeur est condensée et la chaleur latente ainsi libérée est utilisée comme source de chaleur dudit rebouilleur;

(e) adresser le liquide de composants à bas point d'ébullition condensé dans ledit rebouilleur et la vapeur de composants à bas point d'ébullition non-condensée dans ledit rebouilleur, à un réservoir (18) de séparation vapeur-liquide, et séparer le liquide condensé de la vapeur non-condensée;

(f) adresser la vapeur non-condensée de composants à bas point d'ébullition, dudit réservoir de séparation audit condenseur (5);

(g) adresser le liquide condensé de composants à bas point d'ébullition provenant dudit réservoir de séparation audit dispositif de préchauffage (16), pour y subir un échange de chaleur avec la vapeur de composants à bas point d'ébullition allant audit compresseur (7);

(h) renvoyer une partie du liquide condensé de composants à bas point d'ébullition condensé dans ledit condenseur et ledit rebouilleur à la partie supérieure de ladite colonne de distillation, et retirer la partie restante dudit liquide condensé de composants à bas point d'ébullition du système de distillation.

2. Procédé selon la revendication 1, comprenant en outre l'étape consistant à adresser une partie de la vapeur comprimée de composants à bas point d'ébullition provenant dudit compresseur (7), audit condenseur (5), la partie restante de ladite vapeur comprimée étant adressée audit rebouilleur (13).

3. Procédé selon l'une des revendications 1 et 2, suivant lequel on utilise lesdits composants à bas point d'ébullition, condensés dans ledit rebouilleur, comme source de chaleur d'un échangeur de chaleur (33), pour maintenir un reflux renvoyé à ladite colonne de distillation à une température déterminée.

4. Procédé selon l'une des revendications 1 à 3, suivant lequel on effectue la commande de l'avancement des opérations en surveillant la température interne en un emplacement à l'intérieur de ladite colonne de distillation (2), en analysant en continu ladite composition de styrène introduite dans ladite colonne de distillation (2) et, sur la base des signaux de données ainsi produits, en réglant la pression dans ledit rebouilleur (13).

5. Procédé selon la revendication 4, comprenant le réglage en cascade de la quantité desdits composants à bas point d'ébullition qui est retirée et/ou le taux du transfert de chaleur audit rebouilleur (13).

6. Procédé selon l'une des revendications 1 à 5, suivant lequel un rebouilleur auxiliaire (31) est prévu pour introduire de la chaleur provenant d'une source extérieure, et suivant lequel on fait démarrer la distillation en chargeant à l'avance ladite colonne de distillation (2) avec les matières brutes pour la distillation ou avec des composants à pas point d'ébullition, en chauffant la charge de ladite colonne de distillation avec ledit rebouilleur auxiliaire, pour faire démarrer ainsi la distillation à l'état de reflux total, et en utilisant du liquide retiré du bas de ladite colonne de distillation pour préchauffer ladite vapeur de composants à bas point d'ébullition introduits dans ledit compresseur (7).

7. Procédé selon la revendication 6, suivant lequel les conditions de température et de pression à l'intérieur de l'appareil de distillation sont amenées approximativement à des niveaux de fonctionnement désirés, avant que l'on ne commence à alimenter en continu ladite colonne de distillation avec ledit mélange de styrènes.

8. Procédé selon l'une des revendications 1 à 7, dans lequel ladite composition de styrène comprend au moins un éthylbenzène et son produit de déshydrogénation en styrène correspondant.

# FIG.1